# EUROPEAN PATENT SPECIFICATION

(11) **EP 4 405 035 B1**
(45) Date of publication and mention of the grant of the patent: **15.04.2026**
(21) Application number: 22821636.2
(22) Date of filing: 30.11.2022
(51) Int. Cl.: A61N 1/04, A61N 1/06, A61N 1/40

(54) **DEVICES AND SYSTEMS FOR APPLYING TUMOR-TREATING FIELDS**
VORRICHTUNGEN UND SYSTEME ZUR ANWENDUNG VON TUMORBEHANDLUNGSFELDERN
DISPOSITIFS ET SYSTÈMES POUR APPLIQUER DES CHAMPS DE TRAITEMENT DE TUMEUR

(30) Priority: 30.11.2021 US 202163284357 P
(43) Date of publication of application: 31.07.2024
(73) Proprietor: NOVOCURE GMBH, 6340 Baar (CH)
(72) Inventor: SPECTOR, Yuval, 4287500 Kfar Monash (IL)
(74) Representative: Boden, Keith McMurray
(86) International application number: PCT/IB2022/061600
(87) International publication number: WO 2023/100103

(56) References cited:
- CN-A- 111 481 823
- US-A1- 2020 171 297
- US-A1- 2021 187 289

## Description

### FIELD

This disclosure relates to devices, systems, and methods for providing Tumor-Treating Fields, and, in particular, to devices, systems, and methods for providing electrode assemblies with replaceable adhesive subassemblies for securing the electrode subassemblies to the body of a subject.

### BACKGROUND

Tumor-Treating Fields (TTFs) can be used to treat various types of cancer. For example, TTFs can be applied to a portion of a body of a subject via one or more electrode/transducer arrays. Typically, the electrode arrays couple to a signal generator that generates the TTFs in the transducer arrays.

US-A-2020/0171297 discloses a transducer array for use in tumor-treating fields therapy which has features that increase its flexibility and adhesion to a patient's skin, including a branching configuration and a correspondingly-branching top covering adhesive-backed layer, and a skin-level adhesive layer is provided to help ensure thorough, lasting adhesion of the transducer array to the patient's skin over the course of treatment.

US-A-2021/0187289 discloses a treatment assembly for providing tumor-treating fields, the treatment assembly comprising an inner layer having an inner surface and an outer surface and defining a plurality of openings extending therethrough, a plurality of plates, each plate being at least partially received within a respective one of the openings of the inner layer, and a cover layer attached to the outer surface of the inner layer.

CN-A-111481823 discloses a device for treating multi-organ tumors based on an electric field, which comprises an electric field generation device for outputting an AC signal with adjustable voltage waveform, amplitude and frequency, a plurality of field energy electrode patches, each including a flexible substrate and a plurality of electrode plate bodies arranged on the substrate in an array mode, and a wearable device having the field energy electrode patches arranged at an inner side thereof.

### SUMMARY

Described herein is a system for delivering tumor-treating fields to a body of a patient according to claim 1.

Also described herein is a method of applying an assembly for delivering tumor-treating fields to a body of a patient according to claim 12.

Embodiments of the invention are described in the dependent claims.

The present invention is defined by the appended claims.

Additional advantages of the invention will be set forth in part in the description that follows, and in part will be obvious from the description, or may be learned by practice of the invention. The advantages of the invention will be realized and attained by means of the elements and combinations particularly pointed out in the appended claims. It is to be understood that both the foregoing general description and the following detailed description are exemplary and explanatory only and are not restrictive of the invention which is defined by the appended claims.

### DESCRIPTION OF THE DRAWINGS

These and other features of the preferred embodiments of the invention will become more apparent in the detailed description in which reference is made to the appended drawings wherein:
FIG. 1 is an exploded view of an assembly for delivering tumor-treating fields to a body of a patient.
FIG. 2 is an exploded view of a replaceable adhesive subassembly of the assembly of FIG. 1.
FIG. 3 is a schematic cross sectional view of the replaceable adhesive subassembly of FIG. 2 taken along the plane 3-3.

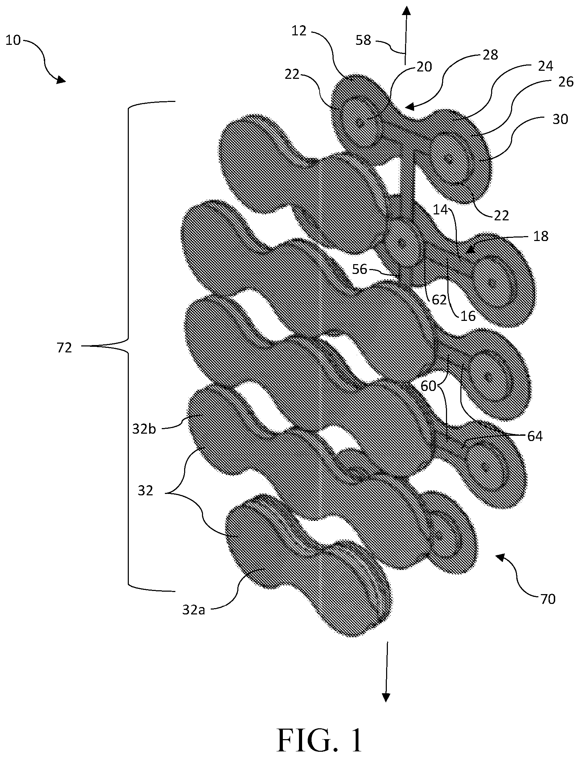

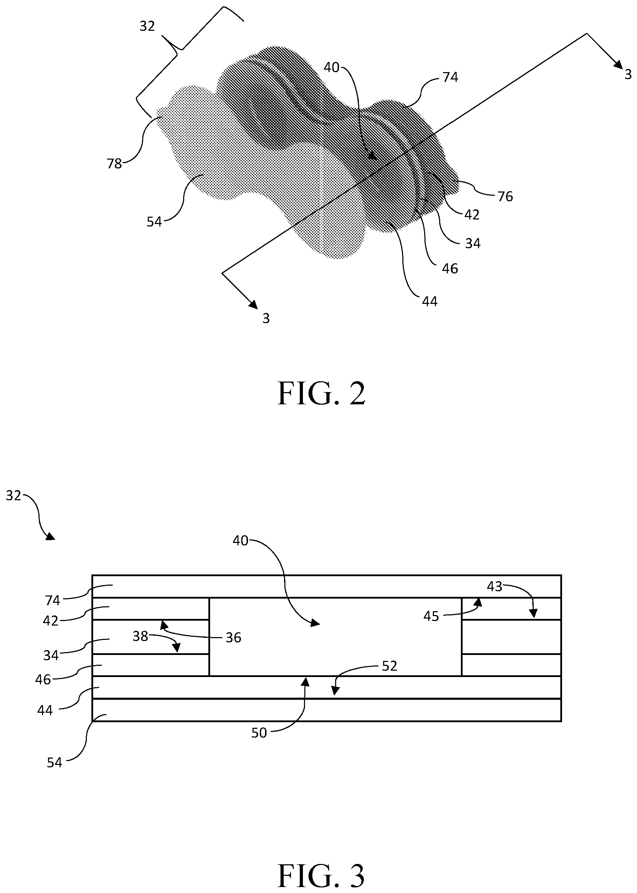

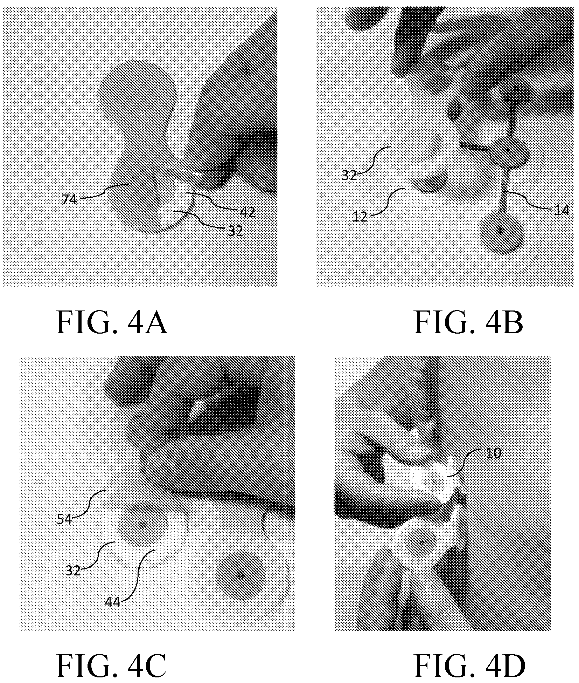

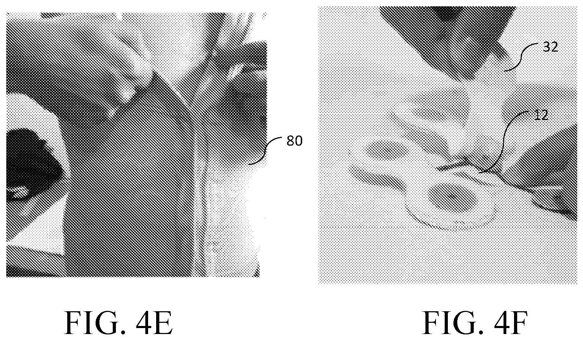

FIG. 4A is a perspective view of a second release liner being removed from a replaceable adhesive subassembly as in FIG. 3. FIG. 4B is a perspective view of the replaceable adhesive subassembly being applied to an electrode subassembly to provide an assembly as in FIG. 1. FIG. 4C is a perspective view of a first release liner being removed from the replaceable adhesive subassembly to expose a biocompatible conductive adhesive. FIG. 4D is a perspective view of a user applying the assembly to a body. FIG. 4E is a perspective view showing application of a vest over the assembly. FIG. 4F is a perspective view of a user removing the replaceable adhesive subassembly from the electrode subassembly.
FIG. 5 is a block diagram of an apparatus for delivering TTFields, in accordance with embodiments disclosed herein.

### DETAILED DESCRIPTION

Tumor-Treating Fields (ITFs) can be used to treat various types of cancer. For example, TTFs can be applied to a portion of a body of a subject via one or more electrode/transducer arrays. Typically, the electrode arrays couple to a signal generator that generates the TTFs in the transducer arrays. Conventionally, a hydrogel is provided on the skin-facing portion of the electrodes for conducting electrical signals to the body of the subject. However, the hydrogel can have a short life, after which the electrode array must be disposed of. What is needed is a way to refresh, replenish or replace the adhesive layer without discarding the electrodes along with the whole electrode array, which is costly. The invention disclosed herein addresses this, as well as other, important subject matters.

The present invention now will be described more fully hereinafter with reference to the accompanying drawings, in which some, but not all embodiments of the invention are shown. Indeed, this invention may be embodied in many different forms and should not be construed as limited to the embodiments set forth herein; rather, it is to be understood that the invention is not to be limited to the specific embodiments disclosed and that the present invention is defined by the appended claims. Although specific terms are employed herein, they are used in a generic and descriptive sense only and not for purposes of limitation.

As used herein the singular forms "a," "an," and "the" include plural referents unless the context clearly dictates otherwise. For example, use of the term "an electrode" or "a replaceable adhesive subassembly" can refer to one or more of such electrodes or replaceable adhesive subassemblies, and so forth.

All technical and scientific terms used herein have the same meaning as commonly understood to one of ordinary skill in the art to which this invention belongs unless clearly indicated otherwise.

Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about" it will be understood that the particular value forms another aspect. It will be further understood that the endpoints of each of the ranges are significant both in relation to the other endpoint, and independently of the other endpoint. Optionally, in some aspects, when values are approximated by use of the antecedent "about," it is contemplated that values within up to 15%, up to 10%, up to 5%, or up to 1% (above or below) of the particularly stated value can be included within the scope of those aspects. Similarly, in some optional aspects, when values are approximated by use of the terms "substantially" or "generally," it is contemplated that values within up to 15%, up to 10%, up to 5%, or up to 1% (above or below) of the particular value can be included within the scope of those aspects. When used with respect to an identified property or circumstance, "substantially" or -"generally" can refer to a degree of deviation that is sufficiently small so as to not measurably detract from the identified property or circumstance, and the exact degree of deviation allowable may in some cases depend on the specific context.

As used herein, the terms "optional" or "optionally" mean that the subsequently described event or circumstance may or may not occur, and that the description includes instances where said event or circumstance occurs and instances where it does not.

As used herein, the term "at least one of" is intended to be synonymous with "one or more of." For example, "at least one of A, B and C" explicitly includes only A, only B, only C, and combinations of each.

The word "or" as used herein means any one member of a particular list and, except where otherwise indicated, can, in alternative aspects, also include any combination of members of that list.

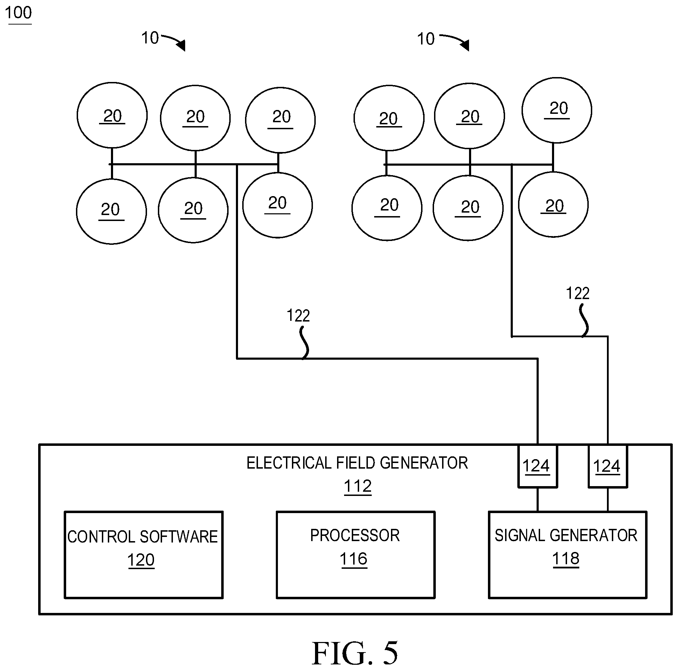

FIG. 5 shows an example apparatus 100 for electrotherapeutic treatment. Generally, the apparatus 100 may be a portable battery or power supply operated device that produces alternating electrical fields within the body by means of transducer arrays or other electrodes. The apparatus 100 may comprise an electrical field generator 112 and one or more electrode (e.g., transducer) arrays (e.g., assemblies 10), each comprising a plurality of electrodes 20. The apparatus 100 may be configured to generate tumor treating fields (TTFields), for example, at frequencies in the range of from about 50 kHz to about 1 MHz, such as from 50 kHz to about 500 kHz (e.g., at about 150 kHz for one tumor cell type, and/or at about 300 kHz for a different tumor cell type), via the electrical field generator 112, and deliver the TTFields to an area of the body through the one or more electrode arrays 10. The electrical field generator 112 may be a battery and/or power supply operated device.

The electrical field generator 112 may comprise a processor 116 in communication with a signal generator 118. The electrical field generator 112 may comprise control software 120 configured for controlling the performance of the processor 116 and the signal generator 118.

The signal generator 118 may generate one or more electric signals in the shape of waveforms or trains of pulses. The signal generator 118 may be configured to generate an alternating voltage waveform, for example, at frequencies in the range from about 50 kHz to about 500 kHz (preferably from about 100 kHz to about 300 kHz) (e.g., the TTFields). The voltages are such that the electrical field intensity in tissue to be treated is typically in the range of about 0.1 V/cm to about 10 V/cm.

One or more outputs 124 of the electrical field generator 112 may be coupled to one or more conductive leads 122 that are attached at one end thereof to the signal generator 118. The opposite ends of the conductive leads 122 are connected to the one or more electrode arrays 10 that are activated by the electric signals (e.g., waveforms). The conductive leads 122 may comprise standard isolated conductors with a flexible metal shield and may be grounded to prevent the spread of the electrical field generated by the conductive leads 122. The one or more outputs 124 may be operated sequentially. Output parameters of the signal generator 118 may comprise, for example, an intensity of the field, a frequency of the waves (e.g., treatment frequency), and a maximum allowable temperature of the one or more electrode arrays 10. The output parameters may be set and/or determined by the control software 120 in conjunction with the processor 116. After determining a desired (e.g., optimal) treatment frequency, the control software 120 may cause the processor 116 to send a control signal to the signal generator 118 that causes the signal generator 118 to output the desired treatment frequency to the one or more electrode arrays 10. It is further contemplated that the control software 120 can cause the processor 116 to shift or change the direction of TTFields or otherwise adjust the properties of TTFields in the manner further disclosed herein.

Disclosed herein, in various aspects, and with reference to FIGS. 1-3, is an assembly 10 for delivering tumor-treating fields to a body of a patient. The assembly 10 can comprise an electrode subassembly 12 comprising a circuitry layer 14 having a skin-facing inner side 16 and an outer side 18 (FIG. 1). The circuitry layer 14 can comprise, for example, flexible circuitry that can permit contour of the circuitry layer 14 to the body of the patient. Optionally, the circuitry layer 14 can comprise a printed circuit board (PCB).

A plurality of electrodes 20 can be disposed on the inner side 16 of the circuitry layer 14 and electrically coupled to the circuitry layer. Each electrode 20 of the plurality of electrodes can have an electrode edge 22. The electrode edge 22 can, for example, trace a perimeter of the electrode 20.

The electrode subassembly 12 can further comprise a covering layer 24 having an inner side 26 and an outer side 28. The inner side 26 of the covering layer 24 can be coupled to the outer side 18 of the circuitry layer 14. For example, the covering layer 24 can be positioned over (e.g., disposed on) all or portions of the circuitry layer 14 with the outer side 18 of the circuitry layer 14 opposing the inner side 26 of the covering layer 24. In some optional aspects, an adhesive can couple the inner covering layer 24 to the circuitry layer. Portions of the inner side 26 of the covering layer 24 can extend beyond (e.g., outwardly from) the circuitry layer 14 and beyond (e.g., outwardly from) the electrode edge 22 of each of the electrodes 20 to define at least one attachment surface 30.

The assembly 10 for delivering tumor-treating fields can further comprise one or more replaceable adhesive subassemblies 32 (FIGS. 1-3). The one or more replaceable adhesive subassemblies 32 can comprise a support layer 34 (FIGS. 2-3) having a first side 36 and a second side 38 (FIG. 3). The support layer 34 can define at least one opening 40 that extends therethrough. Each electrode 20 of the plurality of electrodes can be received within a respective opening 40. In various aspects, the support layer can define 1, 2, 3, 4, 5, 6, 7, 8, 9, 10, or more openings 40 for receiving respective electrodes 20.

A first adhesive 42 (FIGS. 2-3) can be disposed on the first side 36 of the support layer 34 of the adhesive subassembly 32 and can couple the support layer 34 of the adhesive subassembly 32 to the inner side 26 of the covering layer 24 of the electrode subassembly 12 at the at least one attachment surface 30 of the covering layer 24 of the electrode subassembly 12 (FIG. 1). Alternatively, when not coupled to the electrode subassembly 12, the first adhesive 42 may be covered with a release liner 74 (FIGS. 2-3), discussed herein below (and referred to as a second release liner).

In some aspects, the first adhesive 42 can be a nonpermanent adhesive that is configured to permit release from the at least one attachment surface of the covering layer 24 of the electrode subassembly 12. In some aspects, the first adhesive 42 can have a stronger bond to the support layer 34 than to the covering layer 24. For example, the first adhesive can form a bond having a stronger peel strength (e.g., as measured by ASTM D3330 / D3330M) at an interface between the first adhesive 42 and the support layer 34 than at an interface between the first adhesive 42 and the covering layer 24. In this way, the replaceable adhesive subassembly 32 can be removed from the electrode subassembly 12 for replacement of the replaceable adhesive subassembly and reuse of the electrode subassembly 12. In various aspects, the first adhesive can be an acrylic adhesive as known in the art, such as, for example, homopolymer polybutyl acrylate (pBA) or homopolymer polyethylhexyl acrylate (pEHA), or copolymers comprising polymerized units of either BA or EHA or both together, in each case polymerized with or without other monomers which may or may not be acrylic monomers. The term acrylic monomers, as used in the art, refers to esters of acrylic acid, methacrylic acid, itaconic acid, etc., and as used herein additionally refers to the acrylic acid monomers (acrylic acid, methacrylic acid, itaconic acid, etc.).

A biocompatible conductive adhesive 44 can be disposed on the second side 38 of the support layer 34. The biocompatible conductive adhesive 44 can extend across each opening 40 of the one or more openings of the support layer. In this way, the biocompatible conductive adhesive 44 can be applied to the electrode(s) 20 that are received within the openings 40 of the support layer 34. The biocompatible conductive adhesive 44 can be, for example, a hydrogel. One suitable hydrogel is AG603 Hydrogel, which is available from AmGel Technologies, Fallbrook, CA, USA. The hydrogel layer may be a modified hydrogel (for example, having perforations, or recesses, or protrusions, etc., or combination thereof) as disclosed in detail in U.S. Patent Publication No. 2021/0346693 entitled "Conductive Pad Generating Tumor Treating Field and Methods of Production and Use Thereof".

In further aspects, the biocompatible conductive adhesive 44 can be, or comprise, a conductive adhesive composite. In exemplary aspects, the conductive adhesive composite can comprise a dielectric material and conductive particles dispersed within the dielectric material. In some embodiments, at least a portion of the conductive particles define a conductive pathway through a thickness of the conductive adhesive composite. It is contemplated that the conductive particles can be aligned in response to application of an electric field such that the conductive particles undergo electrophoresis. In some aspects, the dielectric material of the conductive adhesive composite is a polymeric adhesive. Optionally, in these aspects, the polymeric adhesive can be an acrylic adhesive as described above. In some aspects, the conductive particles can comprise carbon. Optionally, in these aspects, the conductive particles can comprise graphite powder. Additionally, or alternatively, the conductive particles can comprise carbon flakes. Additionally, or alternatively, the conductive particles can comprise carbon granules. Additionally, or alternatively, the conductive particles can comprise carbon fibers. Additionally, or alternatively, the conductive particles can comprise carbon nanotubes or carbon nanowires. Additionally, or alternatively, the conductive particles can comprise carbon black powder. Additionally, or alternatively, the conductive particles can comprise carbon microcoils. In further aspects, the conductive adhesive composite further comprises a polar material (e.g., a polar salt). The polar salt may be a quaternary ammonium salt, such as a tetra alkyl ammonium salt. Exemplary conductive adhesive composites, as well as methods for making such conductive adhesive composites, are disclosed in U.S. Patent No. 8,673,184 and U.S. Patent No. 9,947,432. In exemplary aspects, the conductive adhesive composite can be a dry carbon/salt adhesive, such as the OMNI-WAVE^{™} adhesive compositions manufactured and sold by FLEXcon^{●} (Spencer, MA, USA). Other conductive adhesive composites may also be suitable, such as, for example, ARcare^{●} 8006 electrically conductive adhesive composition manufactured and sold by Adhesives Research, Inc. (Glen Rock, PA, USA). These same adhesives may also be suitable for use as the first adhesive 42 (FIG. 3) discussed above.

In some optional aspects, the replaceable adhesive subassembly 32 can comprise a second adhesive 46 that is disposed on the second side 38 of the support layer 34. The second adhesive 46 can adhere the biocompatible conductive adhesive 44 to the support layer 34. In further optional aspects, the second adhesive 46 can be omitted.

In some exemplary aspects, the support layer 34 can comprise foam (e.g., polymer foam) or other suitable material that is flexible and provides sufficient thickness to receive the electrodes, such as an elastomer or rubber. In this way, the support layer 34 can be flexible while providing a sufficient thickness to receive the electrodes 20. The support layer 34 can have a thickness that is greater than the thickness of the electrodes 20 or smaller than the thickness of the electrodes 20, but in some exemplary embodiments it has the same or approximately the same thickness.

The biocompatible conductive adhesive 44 can have a first side 50 positioned against the support layer 34 (or against the second adhesive 46, if present) and an opposing second side 52. In some optional aspects, a first release liner 54 can be disposed on the opposing second side 52 of the biocompatible conductive adhesive 44.

In some optional aspects, the circuitry layer 14 (FIG. 1) can comprise a trunk structure 56 that extends along a longitudinal axis 58 and a plurality of branches 60 that extend laterally from the trunk structure. Each of the plurality of branches 60 can have a proximal end 62 that is connected to the trunk structure 56 and an opposing distal end 64 that is outwardly spaced from the trunk structure.

In some aspects, one or more (optionally, all) of the electrodes 20 can be reusable ceramic electrodes. In further aspects, one or more (optionally, all) of the electrodes 20 can be reusable high-dielectric polymer electrodes.

The covering layer 24 of the electrode subassembly 12 can define a first peripheral profile. The support layer 34 can define a second peripheral profile. At least a portion of the first peripheral profile of the covering layer 24 corresponds to at least a portion of the second peripheral profile of the support layer 34. For example, the second peripheral profile can have an arc length that corresponds to at least 50%, at least 60%, at least 70%, at least 80%, at least 90%, or substantially all, or all of the arc length of the first peripheral profile. As used herein, the term "arc length" refers to a length of a curve or perimeter tracing the peripheral profile of a given element. Therefore, the arc length of the first peripheral profile refers to the length of the curve or perimeter that traces the first peripheral profile, and the arc length of the second peripheral profile refers to the length of the curve or perimeter that traces the second peripheral profile.

Optionally, the assembly 10 can comprise a plurality of replaceable adhesive subassemblies 32 that cover respective portions of the covering layer 24. For example, assembly 10 can comprise 2, 3, 4, 5, 6, 7, 8, or more replaceable adhesive subassemblies 32 that form a set of replaceable adhesive subassemblies. A set 72 of replaceable adhesive subassemblies 32 can receive and/or provide biocompatible conductive adhesive 44 to cover each of the electrodes 20 of the electrode subassembly 12. That is, the replaceable adhesive subassemblies 32 of one set 72 can cooperate with the electrode subassembly 12 to form the assembly 10. Optionally, two or more of the plurality of replaceable adhesive subassemblies 32 of a set 72 can be substantially similar or identical to each other. For example, as shown in FIG. 1, the set 72 of the plurality of replaceable adhesive subassemblies 32 can comprise two of a first replaceable adhesive subassembly 32a and three of a second replaceable adhesive subassembly 32b that is different from the first replaceable adhesive subassembly. Different replaceable adhesive subassemblies 32 can differ in peripheral profile, number of openings 40 for receiving electrodes 20, etc. For example, the first replaceable adhesive subassembly 32a can be configured to receive and/or provide biocompatible conductive adhesive to cover two electrodes, and the second replaceable adhesive subassembly 32b can be configured to receive and/or provide biocompatible conductive adhesive to cover three electrodes. In some embodiments (not shown in the Figures), a replaceable adhesive subassembly 32 can be configured to receive and/or provide biocompatible conductive adhesive to cover a single electrode. In further aspects, all of the replaceable adhesive subassemblies 32 of an assembly 10 can be identical. In further optional aspects, the assembly 10 can comprise a single replaceable adhesive subassembly 32. Thus, in some optional aspects, a set 72 can have only one replaceable adhesive subassembly 32.

A system 70 (FIG. 1) can comprise an electrode subassembly 12 as disclosed herein and at least one replaceable adhesive subassembly 32. In some aspects, the system 70 can comprise a plurality of replaceable adhesive subassemblies 32. In some optional aspects, the system 70 can comprise a first set 72 of replaceable adhesive subassemblies 32 as well as one or more sets of replaceable adhesive subassemblies that can be used as replacement adhesive subassemblies to replace used replaceable adhesive subassemblies. Accordingly, the replacement adhesive subassemblies can be structurally similar to, or identical to, the replaceable adhesive subassemblies 32 as described herein. For example, it is contemplated that the replacement adhesive subassemblies can comprise a support layer having a first side and a second side, the support layer defining at least one opening that is configured to receive therein a respective electrode of the electrode subassembly. A first adhesive can be disposed on the first side of the support layer of the replacement adhesive subassembly and can be configured to couple the support layer to the covering layer of the electrode subassembly at the at least one attachment surface of the covering layer. A biocompatible conductive adhesive can be disposed on the second side of the support layer.

As disclosed above, it is contemplated that a plurality of replaceable adhesive subassemblies 32 can cover respective portions of the covering layer 24 so that a set 72 receives and/or provides biocompatible conductive adhesive to cover each of the electrodes 20 of the electrode subassembly 12. For example, as illustrated in FIG. 1, a set can comprise two replaceable adhesive subassembly sets 32a and three replaceable adhesive subassembly sets 32b. It is contemplated that the system 70 can comprise a plurality of sets 72 of replaceable adhesive subassemblies 32, wherein each set is configured to receive and/or provide biocompatible conductive adhesive to cover each of the electrodes 20 of the electrode subassembly 12. In some aspects, the system 70 can comprise a plurality (for example, 10, 20, 30, 40, or more) of replacement adhesive subassemblies. For example, the system 70 can comprise one or more sets of replaceable adhesive subassemblies 72 for use as replacement adhesive subassemblies. The number of component replaceable adhesive subassemblies 32 or the number of sets of replaceable adhesive subassemblies 72 for use as replacement adhesive subassemblies can vary depending on how often they may need replacing and the projected duration of the treatment. The adhesive subassemblies may need replacing every day or every 2-3 days.

The first adhesive 42 can have a first side 43 that faces the support layer 34 and an opposed second side 45 (FIG. 3). In some aspects, a second release liner 74 can be disposed on (e.g., positioned against) the opposed second side 45 of the first adhesive 42. In some aspects, at least a portion of the second release liner 74 can extend outwardly from the second peripheral profile of the support layer 34 to provide a tab 76 (FIG. 2) for removing the second release liner from the replaceable adhesive subassembly 32. Similarly, at least a portion of the first release liner 54 can extend outwardly from the second peripheral profile of the support layer 34 to provide a tab 78 (FIG. 2) for removing the first release liner from the rest of the replaceable adhesive subassembly 32.

Referring also to FIGS. 4A-4F and 5, a method can comprise applying an assembly 10 to a body of a patient. Using the electrode subassembly 12, tumor-treating fields can be delivered to the patient. For example, as described herein, the electrode subassembly 12 of the assembly 10 can be in electrical communication with the electrical field generator 112, and electrical field generator can cause the electrodes to generate TTFields therebetween.

In some aspects, the assembly 10 can be provided in a pre-assembled manner with the replaceable adhesive subassembly (or subassemblies) 32 attached to the electrode subassembly 12. In further aspects, the assembly 10 can be prepared. For example, as shown in FIG. 4A, the second release liner 74 can be removed from the first adhesive 42 of the replaceable adhesive subassembly 32 to expose the first adhesive 42. Referring to FIG. 4B, the replaceable adhesive subassembly 32 (without the second release liner 74) can be applied to the electrode subassembly 12 so that the electrodes are received within the openings 40. The first adhesive 42 can releasably attach the replaceable adhesive subassembly 32 to the attachment surface 30 of the electrode subassembly 12 so that the replaceable adhesive subassembly 32 can subsequently be removed from the electrode subassembly. The application of additional replaceable adhesive subassemblies 32 can be repeated until a set 72 of replaceable adhesive subassemblies cover each of the electrodes 20, thereby providing the assembly 10. Referring to FIG. 4C, the first release liner 54 can be removed from the biocompatible conductive adhesive 44 of the assembly 10 to expose the biocompatible conductive adhesive. Referring to FIG. 4D, the assembly 10 (without the first release liner 54) can be applied to the body of the patient with the biocompatible conductive adhesive 44 positioned against the skin of the patient. Referring to FIG. 4E, optionally, a vest 80 can be positioned over the assembly 10. The vest 80 can resiliently and elastically expand to receive at least a portion of the body (e.g., a torso) of the patient and compress the assembly 10 against the patient.

The assembly 10 can be removed (e.g., after a period of treatment of tumor-treating fields using the assembly 10) from the body of the patient. Referring to FIG. 4F, the replaceable adhesive subassemblies 32 can be removed, and, following the foregoing steps as above, one or more replacement adhesive subassemblies can replace the removed replaceable adhesive subassemblies 32 to provide another assembly 10 that can re-use the electrode subassembly 12. Said assembly 10 (with the replaced replaceable adhesive subassemblies) can then be used on the same patient or on a different patient to deliver TTFields.

As discussed above, a system for delivering tumor-treating fields to a body of a patient may comprise an assembly, such as an assembly resulting from the combination of the electrode subassembly/subassemblies discussed herein with the replaceable adhesive subassembly/subassemblies discussed herein. In some aspects, the system may comprise, or may take the form of, a kit comprising such subassemblies and/or replacement subassemblies or components thereof in any number. For example, the kit may contain or comprise a plurality of one or more such components, such as, for example, replacement adhesive subassemblies configured to cover any number, or all, of the electrodes of the electrode subassembly. For example, in various aspects, the replacement adhesive subassemblies can be configured to cover one electrode, or two electrodes, or three electrodes, or a greater number of electrodes. In some optional aspects, a single replacement adhesive subassembly can be configured to cover all of the electrodes of the electrode subassembly. In some aspects, the kit need not comprise an electrode subassembly. For example, in some aspects, the kit can include only a plurality of adhesive subassemblies as disclosed herein.

Although the foregoing invention has been described in some detail by way of illustration and example for purposes of clarity of understanding, certain changes and modifications may be practiced within the scope of the invention which is defined by the appended claims.

## Claims

1. A system for delivering tumor-treating fields to a body of a patient, the system comprising:
an electrode subassembly (12) comprising:
a circuitry layer (14) having a skin-facing, inner side (16) and an outer side (18);
a plurality of electrodes (20) disposed on the inner side (16) of the circuitry layer (14) and electrically coupled thereto, wherein each of the electrodes (20) has an electrode edge (22); and
a covering layer (24) having an inner side (26) and an outer side (28), wherein the inner side (26) of the covering layer (24) is disposed on the outer side (18) of the circuitry layer (14), and portions of the covering layer (24) extend beyond the circuitry layer (14) and the electrode edge (22) of each of the electrodes (20) to define at least one attachment surface (30); and
at least one replaceable adhesive subassembly (32) comprising:
a support layer (34) having a first side (36) and a second side (38), wherein the support layer (34) defines at least one opening (40) that is configured to receive therein a respective one of the electrodes (20);
a first adhesive (42) that is disposed on the first side (36) of the support layer (34), wherein the first adhesive (42) is configured to couple the support layer (34) to the inner side (26) of the covering layer (24) at the at least one attachment surface (30) thereof and being a nonpermanent adhesive that is configured to permit release from the at least one attachment surface (30) of the covering layer (24); and
a biocompatible conductive adhesive (44) that is disposed on the second side (38) of the support layer (34).

2. The system of claim 1, wherein the at least one replaceable adhesive subassembly (32) further comprises:
a second adhesive (46) that is disposed on the second side (38) of the support layer (34) and adheres the biocompatible conductive adhesive (44) to the support layer (34).

3. The system of claim 1, wherein the support layer (34) comprises foam.

4. The system of claim 1, wherein the biocompatible conductive adhesive (44) has a first side (50) facing the support layer (34) and an opposing, second side (52), and the at least one replaceable adhesive subassembly (32) further comprises:
a first release liner (54) disposed on the second side (52) of the biocompatible conductive adhesive (44).

5. The system of claim 1, wherein the biocompatible conductive adhesive (44) comprises a hydrogel.

6. The system of claim 1, wherein the biocompatible conductive adhesive (44) comprises a conductive adhesive composite.

7. The system of claim 1, wherein the electrodes (20) comprise at least one reusable ceramic electrode.

8. The system of claim 1, wherein the electrodes (20) comprise at least one reusable, high-dielectric polymer electrode.

9. The system of claim 1, wherein the first adhesive (42) has a first side (43) that faces the support layer (24) and an opposing, second side (45), and the at least one replaceable adhesive subassembly (32) further comprises:
a second release liner (74) positioned against the second side (45) of the first adhesive (42).

10. The system of claim 1, comprising:
a plurality of replaceable adhesive subassemblies (32), wherein one or more of the replaceable adhesive subassemblies (32) comprises a plurality of replaceable adhesive subassemblies (32a, 32b), and each replacement adhesive subassembly (32a, 32b) comprises:
a support layer (34) having a first side (36) and a second side (38), wherein the support layer (34) defines at least one opening (40) that is configured to receive therein a respective electrode (20);
a first adhesive (42) that is disposed on the first side (36) of the support layer (34) and configured to couple the support layer (34) to the covering layer (24) at the at least one attachment surface (30) thereof; and
a biocompatible conductive adhesive (44) that is disposed on the second side (38) of the support layer (34).

11. A method, comprising:
applying, to a patient, an assembly comprising:
an electrode subassembly (12) comprising:
a circuitry layer (14) having an inner side (16) and an outer side (18);
a plurality of electrodes (20) disposed on the inner side (16) of the circuitry layer (14) and electrically coupled thereto, wherein each of the electrodes (20) has an electrode edge (22); and
a covering layer (24) having an inner side (26) and an outer side (28), wherein the inner side (26) of the covering layer (24) is disposed on the outer side (18) of the circuitry layer (14), and portions of the inner side (26) of the covering layer (24) extend beyond the circuitry layer (14) and the electrode edge (22) of each of the electrodes (20) to define at least one attachment surface (30); and
at least one replaceable adhesive subassembly (32) comprising:
a support layer (34) having a first side (36) and a second side (38), wherein the support layer (34) defines at least one opening (40), and each of the electrodes (20) is received within a respective one of the at least one opening (40);
a first adhesive (42) that is disposed on the first side (36) of the support layer (34) and couples the support layer (34) to the inner side (26) of the covering layer (24) at the at least one attachment surface (30) thereof, wherein the first adhesive (42) is a nonpermanent adhesive that is configured to permit release from the at least one attachment surface (30) of the covering layer (24); and
a biocompatible conductive adhesive (44) that is disposed on the second side (38) of the support layer (34).

12. The method of claim 11, further comprising:
removing one or more of the at least one replaceable adhesive subassembly (32) from the electrode subassembly (12); and
applying one or more replacement adhesive subassemblies (32) to the electrode subassembly (12), wherein each replacement adhesive subassembly (32) comprises:
a support layer (34) having a first side (36) and a second side (38), wherein the support layer (34) defines at least one opening (40) that is configured to receive therein a respective one of the electrodes (20);
a first adhesive (42) that is disposed on the first side (36) of the support layer (34), wherein the first adhesive (42) is configured to couple the support layer (34) to the inner side (26) of the covering layer (24) at the at least one attachment surface (30) thereof; and
a biocompatible conductive adhesive (44) that is disposed on the second side (38) of the support layer (34).

13. The method of claim 12, further comprising:
prior to the removing one or more of the at least one replaceable adhesive subassembly (32) from the electrode subassembly (12), removing the assembly from the patient; and
applying, to the patient, the one or more replacement adhesive subassemblies (32) with the electrode subassembly (12) attached thereto.

14. The method of claim 13, wherein the biocompatible conductive adhesive (44) of each replacement adhesive subassembly (32) has a first side (50) that faces the support layer (34) and an opposing, second side (52), and a first release liner (54) is positioned against the second side (52) of the biocompatible conductive adhesive (44), and the first adhesive (42) of each replacement adhesive subassembly (32) has a first side (43) positioned against the support layer (34) and an opposing, second side (45), and a second release liner (74) is disposed on the second side (45) of the first adhesive (42), and further comprising:
removing the second release liner (74) prior to applying the replacement adhesive subassembly (32) to the electrode subassembly (12); and
removing the first release liner (54) prior to applying, to the patient, the one or more replacement adhesive subassemblies (32) with the electrode subassembly (12) attached thereto.

## Patentansprüche

1. System zur Abgebung von Tumorbehandlungsfeldern an einen Körper eines Patienten, wobei das System umfasst:
eine Elektrodenunterbaugruppe (12), umfassend:
eine Schaltungsschicht (14), die eine der Haut zugewandten Innenseite (16) und eine Außenseite (18) aufweist;
eine Vielzahl von Elektroden (20), die auf der Innenseite (16) der Schaltungsschicht (14) angeordnet und elektrisch mit dieser verbunden sind, wobei jede der Elektroden (20) eine Elektrodenkante (22) aufweist; und
eine Deckschicht (24), die eine Innenseite (26) und eine Außenseite (28) aufweist, wobei die Innenseite (26) der Deckschicht (24) auf der Außenseite (18) der Schaltungsschicht (14) angeordnet ist und Abschnitte der Deckschicht (24) über die Schaltungsschicht (14) und die Elektrodenkante (22) jeder der Elektroden (20) hinausragen, um mindestens eine Haftfläche (30) zu definieren; und
mindestens eine austauschbare Klebeunterbaugruppe (32), umfassend:
eine Trägerschicht (34), die eine erste Seite (36) und eine zweite Seite (38) aufweist, wobei die Trägerschicht (34) mindestens eine Öffnung (40) definiert, die zur Aufnahme einer jeweiligen der Elektroden (20) darin konfiguriert ist;
einen ersten Klebstoff (42), der auf der ersten Seite (36) der Trägerschicht (34) angeordnet ist, wobei der erste Klebstoff (42) so konfiguriert ist, dass er die Trägerschicht (34) mit der Innenseite (26) der Deckschicht (24) an deren mindestens einer Haftfläche (30) verbindet und ein nicht permanenter Klebstoff ist, der so konfiguriert ist, dass er ein Ablösen von der mindestens einen Haftfläche (30) der Deckschicht (24) ermöglicht; und
einen biokompatiblen leitfähigen Klebstoff (44), der auf der zweiten Seite (38) der Trägerschicht (34) angeordnet ist.

2. System nach Anspruch 1, wobei die mindestens eine austauschbare Klebeunterbaugruppe (32) weiter Folgendes umfasst:
einen zweiten Klebstoff (46), der auf der zweiten Seite (38) der Trägerschicht (34) angeordnet ist und den biokompatiblen leitfähigen Klebstoff (44) an der Trägerschicht (34) klebt.

3. System nach Anspruch 1, wobei die Trägerschicht (34) Schaumstoff umfasst.

4. System nach Anspruch 1, wobei der biokompatible leitfähige Klebstoff (44) eine erste Seite (50), die der Trägerschicht (34) zugewandt ist, und eine gegenüberliegende zweite Seite (52) aufweist, und die mindestens eine austauschbare Klebeunterbaugruppe (32) weiter Folgendes umfasst:
eine erste Trennfolie (54), die auf der zweiten Seite (52) des biokompatiblen leitfähigen Klebstoffs (44) angeordnet ist.

5. System nach Anspruch 1, wobei der biokompatible leitfähige Klebstoff (44) ein Hydrogel umfasst.

6. System nach Anspruch 1, wobei der biokompatible leitfähige Klebstoff (44) einen leitfähigen Klebstoffverbund umfasst.

7. System nach Anspruch 1, wobei die Elektroden (20) mindestens eine wiederverwendbare Keramikelektrode umfassen.

8. System nach Anspruch 1, wobei die Elektroden (20) mindestens eine wiederverwendbare Polymerelektrode mit hoher Dielektrizitätskonstante umfassen.

9. System nach Anspruch 1, wobei der erste Klebstoff (42) eine erste Seite (43), die der Trägerschicht (24) zugewandt ist, und eine gegenüberliegende zweite Seite (45) aufweist, und die mindestens eine austauschbare Klebeunterbaugruppe (32) weiter Folgendes umfasst:
eine zweite Trennfolie (74), die an der zweiten Seite (45) des ersten Klebstoffs (42) anliegt.

10. System nach Anspruch 1, umfassend:
eine Vielzahl austauschbarer Klebeunterbaugruppen (32), wobei eine oder mehrere der austauschbaren Klebebaugruppen (32) eine Vielzahl austauschbarer Klebeunterbaugruppen (32a, 32b) umfassen und jede austauschbare Klebeunterbaugruppe (32a, 32b) Folgendes umfasst:
eine Trägerschicht (34), die eine erste Seite (36) und eine zweite Seite (38) aufweist, wobei die Trägerschicht (34) mindestens eine Öffnung (40) definiert, die zur Aufnahme einer jeweiligen Elektrode (20) darin ausgebildet ist;
einen ersten Klebstoff (42), der auf der ersten Seite (36) der Trägerschicht (34) angeordnet ist und so konfiguriert ist, dass er die Trägerschicht (34) an der mindestens einen Haftfläche (30) mit der Deckschicht (24) verbindet; und
einen biokompatiblen leitfähigen Klebstoff (44), der auf der zweiten Seite (38) der Trägerschicht (34) angeordnet ist.

11. Verfahren, umfassend:
Aufbringen einer Anordnung auf einen Patienten, das Folgendes umfasst:
eine Elektrodenunterbaugruppe (12), umfassend:
eine Schaltungsschicht (14), die eine Innenseite (16) und eine Außenseite (18) aufweist;
eine Vielzahl von Elektroden (20), die auf der Innenseite (16) der Schaltungsschicht (14) angeordnet und elektrisch mit dieser verbunden sind, wobei jede der Elektroden (20) eine Elektrodenkante (22) aufweist; und
eine Deckschicht (24), die eine Innenseite (26) und eine Außenseite (28) aufweist, wobei die Innenseite (26) der Deckschicht (24) auf der Außenseite (18) der Schaltungsschicht (14) angeordnet ist und Abschnitte der Innenseite (26) der Deckschicht (24) über die Schaltungsschicht (14) und die Elektrodenkante (22) jeder der Elektroden (20) hinausragen, um mindestens eine Haftfläche (30) zu definieren; und
mindestens eine austauschbare Klebeunterbaugruppe (32), umfassend:
eine Trägerschicht (34), die eine erste Seite (36) und eine zweite Seite (38) aufweist, wobei die Trägerschicht (34) mindestens eine Öffnung (40) definiert und jede der Elektroden (20) in einer jeweiligen der mindestens einen Öffnung (40) aufgenommen ist;
einen ersten Klebstoff (42), der auf der ersten Seite (36) der Trägerschicht (34) angeordnet ist und die Trägerschicht (34) mit der Innenseite (26) der Deckschicht (24) an deren mindestens einer Haftfläche (30) verbindet, wobei der erste Klebstoff (42) ein nicht permanenter Klebstoff ist, der so ausgebildet ist, dass er ein Ablösen von der mindestens einen Haftfläche (30) der Deckschicht (24) ermöglicht; und
einen biokompatiblen leitfähigen Klebstoff (44), der auf der zweiten Seite (38) der Trägerschicht (34) angeordnet ist.

12. Verfahren nach Anspruch 11, weiter umfassend:
Entfernen einer oder mehrerer der mindestens einen austauschbaren Klebeunterbaugruppe (32) von der Elektrodenunterbaugruppe (12); und
Aufbringen einer oder mehrerer Ersatzklebeunterbaugruppen (32) auf die Elektrodenunterbaugruppe (12), wobei jede Ersatzklebeunterbaugruppe (32) Folgendes umfasst:
eine Trägerschicht (34), die eine erste Seite (36) und eine zweite Seite (38) aufweist, wobei die Trägerschicht (34) mindestens eine Öffnung (40) definiert, die zur Aufnahme einer jeweiligen der Elektroden (20) darin konfiguriert ist;
einen ersten Klebstoff (42), der auf der ersten Seite (36) der Trägerschicht (34) angeordnet ist, wobei der erste Klebstoff (42) so ausgebildet ist, dass er die Trägerschicht (34) mit der Innenseite (26) der Deckschicht (24) an deren mindestens einer Haftfläche (30) verbindet; und
einen biokompatiblen leitfähigen Klebstoff (44), der auf der zweiten Seite (38) der Trägerschicht (34) angeordnet ist.

13. Verfahren nach Anspruch 12, weiter umfassend:
vor dem Entfernen einer oder mehrerer der mindestens einen austauschbaren Klebeunterbaugruppe (32) von der Elektrodenbaugruppe (12), Entfernen der Anordnung von den Patienten; und
Aufbringen der einen oder mehrerer Ersatzklebeunterbaugruppen (32) mit der daran befestigten Elektrodenunterbaugruppe (12) auf den Patienten.

14. Verfahren nach Anspruch 13, wobei der biokompatible leitfähige Klebstoff (44) jeder Ersatzklebeunterbaugruppe (32) eine erste Seite (50), die der Trägerschicht (34) zugewandt ist, und eine gegenüberliegende zweite Seite (52) aufweist, und eine erste Trennfolie (54) an der zweiten Seite (52) des biokompatiblen leitfähigen Klebstoffs (44) anliegt, und der erste Klebstoff (42) jeder Ersatzklebeunterbaugruppe (32) eine erste Seite (43), die an der Trägerschicht (34) anliegt, und eine gegenüberliegende zweite Seite (45) aufweist, und eine zweite Trennfolie (74) auf der zweiten Seite (45) des ersten Klebstoffs (42) angeordnet ist, und weiter umfassend: Entfernen der zweiten Trennfolie (74) vor dem Aufbringen der Ersatzklebeunterbaugruppe (32) auf die Elektrodenunterbaugruppe (12); und Entfernen der ersten Trennfolie (54) vor dem Aufbringen der einen oder mehreren Ersatzklebeunterbaugruppen (32) mit der daran befestigten Elektrodenunterbaugruppe (12) auf den Patienten.

## Revendications

1. Système de délivrance de champs de traitement de tumeur à un corps d'un patient, le système comprenant :
un sous-ensemble d'électrodes (12) comprenant :
une couche de circuits (14) présentant une face interne orientée vers la peau (16) et une face externe (18) ;
une pluralité d'électrodes (20) disposées sur la face interne (16) de la couche de circuits (14) et couplées électriquement à celle-ci, dans lequel chacune des électrodes (20) présente un bord d'électrode (22) ; et
une couche de revêtement (24) présentant une face interne (26) et une face externe (28), dans lequel la face interne (26) de la couche de revêtement (24) est disposée sur la face externe (18) de la couche de circuits (14), et des parties de la couche de revêtement (24) s'étendent au-delà de la couche de circuits (14) et du bord d'électrode (22) de chacune des électrodes (20) de façon à définir au moins une surface de fixation (30) ; et
au moins un sous-ensemble d'adhésifs remplaçables (32) comprenant :
une couche de support (34) présentant une première face (36) et une seconde face (38), dans lequel la couche de support (34) définit au moins une ouverture (40) qui est configurée pour recevoir une électrode respective parmi les électrodes (20) ;
un premier adhésif (42) qui est disposé sur la première face (36) de la couche de support (34), dans lequel le premier adhésif (42) est configuré pour coupler la couche de support (34) à la face interne (26) de la couche de revêtement (24) au niveau de la au moins une surface de fixation (30) de celle-ci et est un adhésif non permanent qui est configuré pour permettre un détachement de la au moins une surface de fixation (30) de la couche de revêtement (24) ; et
un adhésif conducteur biocompatible (44) qui est disposé sur la seconde face (38) de la couche de support (34).

2. Système selon la revendication 1, dans lequel le au moins un sous-ensemble d'adhésifs remplaçables (32) comprend en outre :
un second adhésif (46) qui est disposé sur la seconde face (38) de la couche de support (34) et qui colle l'adhésif conducteur biocompatible (44) à la couche de support (34).

3. Système selon la revendication 1, dans lequel la couche de support (34) comprend de la mousse.

4. Système selon la revendication 1, dans lequel l'adhésif conducteur biocompatible (44) présente une première face (50) orientée vers la couche de support (34) et une seconde face à l'opposé (52), et le au moins un sous-ensemble d'adhésifs remplaçables (32) comprend en outre :
une première pellicule de protection (54) disposée sur la seconde face (52) de l'adhésif conducteur biocompatible (44).

5. Système selon la revendication 1, dans lequel l'adhésif conducteur biocompatible (44) comprend un hydrogel.

6. Système selon la revendication 1, dans lequel l'adhésif conducteur biocompatible (44) comprend un composite adhésif conducteur.

7. Système selon la revendication 1, dans lequel les électrodes (20) comprennent au moins une électrode en céramique réutilisable.

8. Système selon la revendication 1, dans lequel les électrodes (20) comprennent au moins une électrode polymère hautement diélectrique réutilisable.

9. Système selon la revendication 1, dans lequel le premier adhésif (42) présente une première face (43) qui est orientée vers la couche de support (24) et une seconde face à l'opposé (45), et le au moins un sous-ensemble d'adhésifs remplaçables (32) comprend en outre :
une seconde pellicule de protection (74) positionnée contre la seconde face (45) du premier adhésif (42).

10. Système selon la revendication 1, comprenant :
une pluralité de sous-ensembles d'adhésifs remplaçables (32), dans lequel un ou plusieurs des sous-ensembles d'adhésifs remplaçables (32) comprennent une pluralité de sous-ensembles d'adhésifs remplaçables (32a, 32b), et chaque sous-ensemble d'adhésifs remplaçables (32a, 32b) comprend :
une couche de support (34) présentant une première face (36) et une seconde face (38), dans lequel la couche de support (34) définit au moins une ouverture (40) qui est configurée pour recevoir une électrode respective (20) ;
un premier adhésif (42) qui est disposé sur la première face (36) de la couche de support (34) et est configuré pour coupler la couche de support (34) à la couche de revêtement (24) au niveau de la au moins une surface de fixation (30) de celle-ci ; et
un adhésif conducteur biocompatible (44) qui est disposé sur la seconde face (38) de la couche de support (34).

11. Procédé, comprenant :
l'application, à un patient, d'un ensemble comprenant :
un sous-ensemble d'électrodes (12) comprenant :
une couche de circuits (14) présentant une face interne (16) et une face externe (18) ;
une pluralité d'électrodes (20) disposées sur la face interne (16) de la couche de circuits (14) et couplées électriquement à celle-ci, dans lequel chacune des électrodes (20) présente un bord d'électrode (22) ; et
une couche de revêtement (24) présentant une face interne (26) et une face externe (28), dans lequel la face interne (26) de la couche de revêtement (24) est disposée sur la face externe (18) de la couche de circuits (14), et des parties de la face interne (26) de la couche de revêtement (24) s'étendent au-delà de la couche de circuits (14) et du bord d'électrode (22) de chacune des électrodes (20) de façon à définir au moins une surface de fixation (30) ; et
au moins un sous-ensemble d'adhésifs remplaçables (32) comprenant :
une couche de support (34) présentant une première face (36) et une seconde face (38), dans lequel la couche de support (34) définit au moins une ouverture (40), et chacune des électrodes (20) est reçue dans une ouverture respective parmi la au moins une ouverture (40) ;
un premier adhésif (42) qui est disposé sur la première face (36) de la couche de support (34) et couple la couche de support (34) à la face interne (26) de la couche de revêtement (24) au niveau de la au moins une surface de fixation (30) de celle-ci, dans lequel le premier adhésif (42) est un adhésif non permanent qui est configuré pour permettre un détachement de la au moins une surface de fixation (30) de la couche de revêtement (24) ; et
un adhésif conducteur biocompatible (44) qui est disposé sur la seconde face (38) de la couche de support (34).

12. Procédé selon la revendication 11, comprenant en outre :
le retrait du sous-ensemble d'électrodes (12) d'un ou plusieurs du au moins un sous-ensemble d'adhésifs remplaçables (32) ; et
l'application d'un ou plusieurs sous-ensembles d'adhésifs remplaçables (32) au sous-ensemble d'électrodes (12), dans lequel chaque sous-ensemble d'adhésifs remplaçables (32) comprend :
une couche de support (34) présentant une première face (36) et une seconde face (38), dans lequel la couche de support (34) définit au moins une ouverture (40) qui est configurée pour recevoir une électrode respective parmi les électrodes (20) ;
un premier adhésif (42) qui est disposé sur la première face (36) de la couche de support (34), dans lequel le premier adhésif (42) est configuré pour coupler la couche de support (34) à la face interne (26) de la couche de revêtement (24) au niveau de la au moins une surface de fixation (30) de celle-ci ; et
un adhésif conducteur biocompatible (44) qui est disposé sur la seconde face (38) de la couche de support (34).

13. Procédé selon la revendication 12, comprenant en outre :
avant de retirer du sous-ensemble d'électrodes (12) un ou plusieurs du au moins un sous-ensemble d'adhésifs remplaçables (32), le retrait de l'ensemble du patient ; et
l'application, au patient, des un ou plusieurs sous-ensembles d'adhésifs remplaçables (32) avec le sous-ensemble d'électrodes (12) fixé à ceux-ci.

14. Procédé selon la revendication 13, dans lequel l'adhésif conducteur biocompatible (44) de chaque sous-ensemble d'adhésifs remplaçables (32) présente une première face (50) qui est orientée vers la couche de support (34) et une seconde face à l'opposé (52), et une première pellicule de protection (54) est positionnée contre la seconde face (52) de l'adhésif conducteur biocompatible (44), et le premier adhésif (42) de chaque sous-ensemble d'adhésifs remplaçables (32) présente une première face (43) positionnée contre la couche de support (34) et une seconde face à l'opposé (45), et une seconde pellicule de protection (74) est disposée sur la seconde face (45) du premier adhésif (42), et comprenant en outre : le retrait de la seconde pellicule de protection (74) avant d'appliquer le sous-ensemble d'adhésifs remplaçables (32) au sous-ensemble d'électrodes (12) ; et le retrait de la première pellicule de protection (54) avant d'appliquer, au patient, les un ou plusieurs sous-ensembles d'adhésifs remplaçables (32) avec le sous-ensemble d'électrodes (12) fixé à ceux-ci.
